# EUROPEAN PATENT SPECIFICATION

(11) **EP 0 959 130 B1**
(45) Date of publication and mention of the grant of the patent: **09.04.2003**
(21) Application number: 98201473.0
(22) Date of filing: 06.05.1998
(51) Int. Cl.: C12N 15/52, C12N 9/34, C12N 15/62

(54) **Cloning and expression of a new glucoamylase gene from endomyces fibuliger**
Klonierung und Expression eines neuen Glukoamylasegens aus endomyces fibuliger
Clonage et expression d'un nouveau gène de glucoamylase d'endomyces fibuliger

(43) Date of publication of application: 24.11.1999
(73) Proprietor: SOCIETE DES PRODUITS NESTLE S.A., 1800 Vevey (CH)
(72) Inventor: Pridmore, Raymond David, 1000 Lausanne 26 (CH); Kochhar, Sunil, 1073 Savigny (CH)
(74) Representative: Becker Kurig Straus

(56) References cited:
- GASPERIK J ET AL: "Amylolytic enzymes produced by Saccharomycopsis fibuligera" BIOLOGIA (BRATISLAVA), vol. 43, no. 8, August 1988, pages 673-679, XP002081218
- HOSTINOVA E ET AL: "The nucleotide sequence of the glucoamylase gene GLA1 from Saccharomycopsis fibuligera KZ" FEMS MICROBIOLOGY LETTERS, vol. 83, no. 1, 15 September 1991, pages 103-108, XP002081219

## Description

The present invention relates to a new recombinant glucoamylase from *Endomyces fibuliger*, a gene encoding this enzyme, recombinant cells expressing this enzyme, and a method for hydrolysing carbohydrate containing materials.

### Background of the invention

The glucoamylase, e.g. 1,4-,1,6-α-D-glucan glucohydrase, is an exoglucosidase that catalyzes the removal of glucose progressively from nonreducing terminals of starch and related polysaccharides. This extracellular enzyme hydrolyzes α-1,4-glucosidic linkages at 30 to 50 times more efficiently than α-1,6-glucosidic linkages (Wong, *In* Food enzymes: structure and mechanism. Chapman and Hall, New York). Other names for this enzyme include γ-amylase, amyloglucosidase, glucamylase, maltase and saccharogenic amylase. Glucoamylase belongs to a group of starch-degrading amylolytic enzymes that include α- and β- amylases, pullulanase and cyclodextrin glycosyltransferase. The end product is almost exclusively glucose, of the β-configuration. The reverse reaction, especially at accumulating glucose concentrations, produces several oligosaccharides such as maltose and isomaltose from glucose.

The industrial production of glucoamylase first started in 1959 where a Japanese corporation produced glucose with *Rhizopus niveus.* In the early stages, the enzyme was produced from wheat bran-koji culture using strains of *Rhizopus* sp. and *Endomyces* sp. Apart from *Rhizopus* sp., commercially-produced glucoamylase is now also available from cultures of *Aspergillus* sp. and used in the saccharification of starch (Amylase Research Society Of Japan, 1988).

The most important application of glucoamylase can be found in the production of crystalline glucose and high glucose syrup consisting of 90 to 97% D-glucose. The glucose produced from starch is used as a substrate for enzymatic production of high fructose syrup. Increasing global demand of glucose and high-fructose syrup production has placed glucoamylase as one of the crucial industrial enzymes (Amylase Research Society of Japan, 1988). Demand on alcohol produced by fermentation is also on the increase, with requirements as alternative energy source, beverage, solvent and raw material in the chemical industry.

The ability of glucoamylase to produce glucose and alcohol from starch-containing raw materials has consequently led to intensive investigations on organisms producing the enzyme.

Glucoamylases have been isolated from micro-organisms such as *Aspergillus* sp., *Rhizopus* sp., *Endomyces* sp., *Monascus* sp., *Mucor rouxianus* and *Penicillium oxalicum*. Multiple forms of the enzyme have been reported in several cases such as in the *Aspergillus* sp., *Rhizopus* sp. and *Endomyces* sp. (Futatsugi *et al*., J. Ferment. Bioeng., 76, 521-523, 1993). Different forms isolated from each strain itself may differ in raw starch digestibility, thermal stability, molecular size, amino acid and carbohydrate composition, and hydrolysis curves on glycogen. The possible advantages for producing multiforms are not clear (Boel *et al*., EMBO J., 3, 1097-1102, 1984). Post-translational modifications and differential mRNA splicing, such as demonstrated in the two glucoamylases of *Aspergillus niger* (Boel *et al*.), are possible mechanisms that generate the different isoforms. It appears that the occurrence of isoenzymes is common in fungi, where the larger of these forms is generally more active in debranching action and stronger in raw starch adsorbtion and degradation. According to Tanaka *et al*. (Agric. Biol. Chem., 50, 1737-1742, 1986), the other forms usually display similar enzymatic properties towards solubilized polysaccharides. Such similar characteristics were reported in the two forms of glucoamylase from *Aspergillus awamori* (Nunberg *et al*., Mol. Cell. Biol. 4, 2306-2315, 1984) and in the four isoforms of *Aspergillus oryzae* (Razzaque and Ueda, J. Ferment. Tech., 56, 296-302, 1978). In a *Rhizopus* sp., it was reported that the three forms have close enzymatic properties such as heat inactivation and susceptibility to extreme pH (Takahashi *et al*., Biochem., 84, 1183-1194, 1978).

Expression and secretion of the enzyme in *S. cerevisiae* have been achieved with glucoamylase-encoding genes from several micro-organisms such as *Rhizopus* sp. (Ashikari *et al*., Agric. Biol. Chem., 50, 957-964, 1986; Tanaka *et al*.) and *Schwanniomyces occidentalis* (Dohmen *et al*., Gene, 95, 111-121, 1990).

In 1961, on his work on extracellular amylases, Hattori reported the production of a glucoamylase from an *Endomyces* sp. (Agric. Biol. Chem., 25, 737-743 and 895-901). In the last decade, research carried out mainly by Japanese and Czechoslovakian researchers suggests the existence of more than one form of the enzyme in *E. fibuliger*. To date, two forms of glucoamylase from *E. fibuliger* have been predominantly reported in the literature, although Gasperik *et al*. (Biologia. Bratisl., 43, 673-680, 1988) obtained three fractions of glucoamylases from an extracellular amylase complex. There was no further report on this third isoenzyme since.

The first report on the isolation of the gene encoding Glucoamylase I was presented by Yamashita *et al.* (Appl. Microbiol. Biotech., 23, 130-133, 1985). From a genomic library of *E. fibuliger* HUT7212, a DNA fragment cloned and transformed into *S. cerevisiae* gave transformants that formed turbid precipitates on soluble-starch (3% w/v) plates. Yamashita *et al.* further analyzed the glucoamylase secreted into culture medium from these transformants. Glucoamylase activity was detected by measuring the amount of glucose produced by reacting dialyzed culture supernatant with soluble starch. The activity was found to be 5.2 units per ml of dialyzed supernatant. One unit of the enzyme activity is defined by the amount of enzyme needed to produce 1 mg of glucose from soluble starch, under the selected reaction conditions. The secreted enzyme from *S. cerevisiae* transformants exhibited identical enzymatic properties, such as dependencies on temperature and pH, as did the parental *E. fibuliger* strain.

Itoh *et al*. (J. Bact., 169, 4171-4176, 1987) continued the cloning work from Yamashita *et al*. The complete nucleotide sequence of the same *E. fibuliger* glucoamylase gene was elucidated. This gene was designated as *GLU1*. The genomic fragment which was previously expressed in *S. cerevisiae* to produce glucoamylase activity was 2,538 nucleotides long, encompassing the 1,564 bp *GLU1* open reading frame and approximately 1,000 nucleotides of flanking DNA. The *GLU1* DNA hybridized to a single 2.1 kb polyadenylated RNA. The ORF corresponds to a peptide of 519 amino acid residues. A hydrophobic segment of 20 amino acids which resembled signal sequences reported in many secretory protein precursors was present in the amino terminal. Amino acid sequence comparison with glucoamylases from *S. cerevisiae, R. niveus, A. niger and Saccharomyces diasticus*, revealed five highly homologous regions. For further characterizations, the glucoamylase from the *S. cerevisiae* transformants was purified by column chromatography. Apart from analyzing glycosylation, Itoh *et al.* also determined its molecular weight by polyacrylamide gel electrophoresis and gel filtration, and detected it to be 55,000.

Enzymatic studies on the second glucoamylase of *E. fibuliger* IFO 0111 was reported by another Japanese group. Separated by column chromatography, two forms of glucoamylase that resembled each other in terms of pH stability and optimum temperature for glucoamylase activity towards soluble starch (40°C ) were reported by Futatsugi *et al.* (J. Ferment. Bioeng. 76, 521-523, 1993). The enzymes were obtained from an *E. fibuliger* culture supernatant, which exhibited glucoamylase activity from its ability to release glucose from hydrolysis of soluble starch. This glucoamylase activity was assayed similarly as by Yamashita *et al*. (1985) and Dohmen *et al.* (1990). The activity of Glucoamylase II was 35% lower than that of Glucoamylase I. Activities of both the purified isoforms were not affected by various cations such as Mg²⁺, Ca²⁺, Fe³⁺, Zn²⁺ and Cu²⁺, and of other reagents such as urea, SDS, EDTA, Triton X-100 and Tween-20. The differences between the two glucoamylases include thermal stability and molecular weight with glucoamylase II (57,000) being larger than glucoamylase I (55,000). Relative amounts of the enzymes depend on growth conditions and duration. Futatsugi *et al*. (1993) has earlier produced glucoamylase in large-scale using 20-*l*, 100-*l*, 200-*l* and 4000-*l* fermentors under aerobic conditions. A list of the characteristics of both these glucoamylases is presented in Table 1 below.

It is interesting to note that another *E. fibuliger* glucoamylase gene was isolated and sequenced by Hostinova *et al*. (Biologia. Bratisl., 45, 301-306, 1990; FEMS Lett., 83, 103-108, 1990, 1991). Designated as *GLA1*, the 2.5 kb genomic DNA fragment from *E. fibuliger* KZ was cloned and expressed in *S. cerevisiae*. Positive transformants were selected by characteristic opaque halo on starch-containing plates, due to the secreted glucoamylase. The entire 2.5 kb fragment was sequenced. When aligned with the *GLU1* ORF nucleotide and its deduced amino acid sequence (Itoh *et al*., 1987), a high 98.5% amino acid sequence identity was observed. Three amino acids in the signal peptide region and eight amino acids in the mature protein were the major differences, apart from differences in the 5'- and 3'-flanking DNA regions. Glucoamylase activity from *GLA1* transformants was detected by the standard assay test, and was found to be three times lower than the glucoamylase from *GLU1* transformants. These two enzymes also differ in molecular weights, and mobility on polyacrylamide gels. These differences could be consequences of the differences in the primary structure of the mature gene product (Hostinova *et al*., 1991). Because of the high amino acid homology, it is possible that the basis of the differences between the *GLA1* and *GLU1* genes is due to the fact that *GLA1* is indeed a *GLU1* that was isolated from another *E. fibuliger* strain. Gasperik and Hostinova (Curr. Microbiol., 27, 11-14, 1993) further studied the glucoamylases from these *GLA1* and *GLU1* transformants. Due to the heterogeneity of glycosylation, the glucoamylases of each gene were isolated and purified in two forms. The two forms of each gene were similar in catalytic and physiochemical characteristics such as pH dependencies and temperature optima, but differed in thermal stability and the capability to renature after heat denaturation.

**Table 1**

| Comparison of some characteristics of purified glucoamylase I and II from *E.fibuliger* IFO 0111 (from Futatsugi *et al*., 1993) | | |
|---|---|---|
| Property | Glucoamylase I | Glucoamylase II |
| Molecular weight | 55,000 | 57,000 |
| Carbohydrate content (by weight) | 16% | 19% |
| Limit of hydrolysis of soluble starch | 85% | 80% |
| *K*ₘ for maltose | 8.3 mM | 2.6 mM |
| pI | 6.1 | 6.5 |
| pH optimum^{a} | 5.5 | 6.0 |
| stability (5h at 37°C) | 5.0-7.0 | 5.0-7.0 |
| Temperature optimum^{b} | 40°C | 40°C |
| stability (*k*_{D}^{c} at 45°C) | 0.0047 min⁻¹ | 0.0021 min⁻¹ |

| | | |
|---|---|---|
| ^{a,b} refers to the optimum pH and temperature, respectively, for glucoamylase activity with soluble starch as substrate | | |
| ^{c} refers to the first order rate constant for inactivation of the glucoamylases Note: the glucoamylase reported by Yamashita *et al*. (1985) has an optimum pH 6.0 and optimum temperature 50°C, whereas both the variant glucoamylases encoded by *GLA1* have an optimum pH 5.4 and optimum temperature 50°C (Gasperik and Hostinova, 1993). | | |

The aim of the invention is to provide the gene of a new glucoamylase from *E. fibuliger*, which enables the construction of new micro-organisms having new hydrolysing specificities against carbohydrate-containing materials.

### Summary of the invention

Accordingly, the present invention has for object a new recombinant glucoamylase from *Endomyces fibuliger* comprising the amino acid sequence starting from one of the amino acids 1-50 up to amino acid 963 of SEQ ID NO:2, or functional derivatives thereof having at least 85% identity.

In a second aspect, the invention has for object a DNA molecule which comprises a *GLA2* gene encoding the enzyme according to the invention.

In a third aspect, the invention provides a cell which expresses the recombinant proteins according of the invention by recombinant technology.

In another aspect, the invention provides a method for producing the enzyme of the invention, comprising cultivating recombinant cells of the invention in a suitable growth medium under conditions that the cells express the enzyme, and optionally isolating the enzyme in the form of a concentrate.

In a last further aspect, the invention provides the use of the recombinant protein or the cells of the invention to hydrolyse carbohydrate-containing materials.

### Detailed description of the invention

Within the following description, the percentages are given by weight except where otherwise stated, and the amino acid or nucleotide sequences referred as "SEQ ID NO:" are always presented in the sequence listing hereafter.

Likewise, the expression *functional derivative of an enzyme* includes all amino acid sequences which differ by substitution, deletion, addition of some amino acids, for instance 1-20 amino acids, but which keep their original activities or functions. The selection of a functional derivative is considered to be obvious to one skilled in the art, since one may easily creates variants of the GLA2 protein (having the amino acid sequence SEQ ID NO:2) by slightly adapting methods known to one skilled in the art, for instance the methods described by Adams *et al*. (EP402450; Genencor), by Dunn *et al.* (Protein Engineering, 2, 283-291, 1988), by Greener *et al.* (Strategies, *7*, 32-34, 1994), and/or by Deng *et al.* (Anal. Biochem, 200, 81, 1992).

A functional derivative protein of the invention is considered as a derivative to another protein, if its sequence is at least 85% identical to the protein, preferably at least 95%, for example. In the context of the present disclosure, the identity is determined by the ratio between the number of amino acids of a derivative sequence which are identical to those of the GLA2 having the amino acid sequence SEQ ID NO:2, and the total number of or amino acids of the said derivative sequence.

In addition, the term "koji" designates the product of the fermentation with a koji mold culture of a mixture of a source of proteins and a source of carbohydrates, especially of a mixture of a leguminous plant or of a cooked oleagginous plant and of a cooked or roasted cereal source, for example of a mixture of soya or cooked beans and of cooked or roasted wheat or rice.

The present invention thus concerns the glucoamylase GLA2 enzyme originating from *Endomyces fibuliger* which comprises the amino acid sequence starting from one of the amino acids 1 - 50 up to amino acid 963 of SEQ ID NO:2, or functional derivatives thereof having at least 85% identity.

This enzyme may be operably fused to any leader peptide faciliting its secretion in a host where the enzyme is expressed, for example the *Endomyces fibuliger* leader peptide having the amino-acid sequence starting from amino acid 1 to one of the amino acids 15 - 30 of SEQ ID NO:2, or any functional derivatives thereof having at least 85% identity.

A *GLA2* gene encoding the GLA2 enzyme according to the invention may at least comprise the coding parts of the nucleotide sequence SEQ ID NO:1, or functional derivatives thereof due to the degeneracy of the genetic code.

A *GLA2* gene may be obtained in substantially purified form by using the method described within the following examples from any strain of *Endomyces fibuliger*, for example strains CNCM I-1991, ATCC 2082, ATCC 2080, ATCC 9947 or ATCC 2088. Alternatively, a *GLA2* gene may be (1) detected also from other genera or species of microoganisms by use of DNA probes derived from the nucleotide sequence SEQ ID NO:1 in a stringent hybridization assay, and (2) recovered by the well known Reverse-PCR method by use of suitable primers, for example primers SEQ ID NO:7 and 8. In a further aspect, a *GLA2* gene may also be *in-vitro* synthesized and then multiplied by using the polymerase chain reaction, for instance.

The DNA molecule according to the invention at least comprises a *GLA2* gene encoding the GLA2 enzyme of the invention. This molecule may be in a form of a vector, i.e. a replicative plasmid or an integrative circular or linearized non replicative plasmid. The DNA molecule thus may comprise, operably linked to the *GLA2* gene, regulatory sequences native to the organism from which derives the gene. Said native regulatory sequences may be the promoter, the terminator, and/or a DNA sequence encoding a signal sequence that originally regulated the secretion of the *GLA2* gene, such as the *Endomyces fibuliger* nucleotide sequence coding for the GLA2 signal peptide. In another embodiment, regulatory sequences may be native sequences that regulate a different gene in the said organism of origin or that regulate a different gene in a foreign organism, for example. A regulatory sequence other than the native regulatory sequence will generally be selected for its high efficiency or desirable characteristic, for example inducibility of a promoter or a sequence encoding a peptide signal which will permit secretion of the protein.

If heterologous expression is preferred, meaning that the genes of the invention are expressed in another organism than the original host (strain, variety, species, genus, family, order, class or division) the regulatory sequences are preferably derived from an organism similar or equal to the expression host. For example, if the expression host is a yeast cell, then the regulatory sequences will be derived from a yeast cell. The promoter suitable for constitutive expression, preferably in a fungal host, may be a promoter from the following genes: glycerolaldhehyde-3-phosphate dehydrogenase, phospho-glycerate kinase, triose phosphate isomerase and acetamidase, for example. Promoter suitable for inducible expression, preferably in a fungal host, may be a promoter from the following genes: endoxylanase IIA, glucoamylase A, cellobiosehydrolase, amylase, invertase, alcohol dehydrogenase and amyloglucosidase. The selection of a desirable regulatory sequence operably linked to a sequence of the invention and capable of directing the expression of the said nucleotide sequence is considered to be obvious tc one skilled in the art.

The DNA molecule according to the invention may also comprise a selection marker to discriminate host cells into which the recombinant DNA material has been introduced from cells that do not comprise the said recombinant material. Such marker genes are, for example in case fungal expression is preferred, the known *ga*-2, *pyr*G, *pyr*4, *pyr*A, *trp*C*, amd*S or *arg*B genes. The DNA molecule may also comprise at least one suitable replication origin. Suitable transformation methods and suitable expression vectors provided with a suitable transcription promoter, suitable transcription termination signals and suitable marker genes for selecting transformed cells are already known in the literature for many organisms including different bacteria, fungal and plant species. In the event fungal expression is required, the expression system described in EP278355 (Novartis) may be thus particularly adapted.

Recombinant cells may be obtained by any method enabling a foreign DNA to be introduced into a cell. Such methods include transformation, electroporation, or any other technique known to those skilled in the art.

The invention thus encompasses a recombinant cell comprising the DNA molecule of the invention, the said cell being able to express the GLA2 of the invention or functional derivatives thereof. These cells may be derived from the group of fungal, yeast, bacterial and plant cells. Preferably, yeast cells are of the genera *Saccharomyces, Kluyveromyces, Hansenula* and *Pichia*, bacterial cells are Gram negative or positive bacteria, i.e. of the genera *Escherichia, Bacillus, Lactobacillus, Lactococcus, Streptococcus* and *Staphylococcus,* plant cells are of the vegetable group, and fungal cells are cells that are tradionnally used for making a koji, such as *Aspergillus, Rhizopus* and/or *Mucor* species, notably *Aspergillus soyae, Aspergillus oryzae* (ATCC 20386), *Aspergillus phoenicis* (ATCC 14332), *Aspergillus niger* (ATCC 1004). *Aspergillus awamori* (ATCC 14331), *Rhizopus oryzae* (ATCC 4858), *Rhizopus oligosporus* (ATCC 22959), *Rhizopus japonicus* (ATCC 8466), *Rhizopus formosaensis*, *Mucor circinelloides* (ATCC 15242), *Mucor japanicus, Penicillium glaucum* and *Penicillium fuscum* (ATCC 10447). Strains referred by an ATCC number are accessible at the American Type Culture Collection, Rockville, Maryland 20852, US. The invention is not limited by such indications which were rather give to enable one skilled in the art to carry out the invention.

Recombinant cells of the invention may comprise the DNA molecule of the invention stably integrated into the chromosome or on a replicative plasmid.

Preferably, functional copies of the *GLA2* gene are integrated at a predefined locus of the chromosomal DNA of the host cell.

Accordingly, in order to operably integrate into the chromosome of prokaryotic cells at least one functional *GLA2* gene which is not fused to any promoter, the DNA molecule of the invention may be integrated by using the process described in EP564966, i.e.,
(1) transforming a host strain organism with a donor plasmid which does not replicate in the host strain, wherein the donor plasmid comprises a vector backbone and a *GLA2* gene of the invention operably integrated, without any promoter, into apart of an operon of the host strain, maintaining the frame and the function of the genomic operon of the host strain; (2) identifying cointegrate transformants in which the complete donor plasmid is integrated into the genomic operon of the host strain; and (3) selecting an integrant transformant from the cointegrate transformants, wherein the genome of the selected integrant transformant does not include the vector backbone of the donor plasmid but does include the *GLA2* gene, which is operably integrated into the conserved genomic operon and which is stably maintained and expressed due to selective pressure on the correct functioning of the essential cistron upon growth in a standard medium.

In a second embodiment, in order to stably integrate into the chromosome of eucaryotic cells only one functional *GLA2* sequence which is fused to a promoter and a terminator which are native to the host organism, DNA molecule of the invention may be integrated by sligthly adapting the process of de Ruiter-Jacobs, Y.M.J.T., Broekhuijsen *et al*. (A gene transfer system based on the homologous *pyr*G gene and efficient expression of bacterial genes in *Aspergillus oryzae*. Curr. Genet. 16: 159-163, 1989), i.e.,
(1) preparing a non-replicative DNA fragment by ligating the *GLA2*, which is operably linked to a promoter and terminator that are native to the host organism, downstream a DNA sequence encoding any essential gene, said essential gene being inactivated by at least a mutation and/or a deletion (this essential gene may be a gene involved in uracil biosynthesis, such as the *pyr*G gene in case *A. oryzae* is used, for example); (2) selecting a host organism containing the essential gene which is however inactivated by another mutation(s) or deletion(s); (3) transforming said host organism with the non-replicative DNA fragment; (4) identifying integrate transformants in which the DNA fragment is integrated so as to restore the native function of the essential gene; (5) selecting an integrate transformant in which only one DNA fragment is integrated.

Progeny of an expression host comprising a DNA molecule according to the invention is also included in the present invention. Accordingly, a preferred embodiment of the invention is directed to a cell comprising a recombinant DNA molecule of the invention in any of the embodiments described above, wherein the said cell is able to integrate the GLA2 into the cell wall or the cell membrane or secrete the enzymes into the periplasmic space or into the culture medium. The secreting route to be followed by the recombinant protein according to the invention will depend on the selected host cell and the composition of the recombinant DNA according to the invention. Most preferably, however, the protein will be secreted into the culture medium. To this end, the cell according to the invention may comprise a recombinant *GLA2* gene further operably linked to a DNA encoding a foreign leader sequence (pre or prepro), for example.

Cells over-expressing the GLA2 of the invention are preferably choosen, especially *Aspergillus* cells. These cells may be obtained by incorporation of the DNA molecule of the present invention in an expression host, said DNA molecule comprising one or more regulatory sequences which serve to increase expression levels of the protein(s) of the invention.

The invention is also directed to a process for producing the GLA2 of the invention comprising, providing recombinant cells according to the invention in a suitable growth medium under conditions that the cells express the GLA2, and optionally isolating the said recombinant protein(s) in the form of a concentrate. The selection of the appropriate medium may be based on the choice of expression host and/or based on the regulatory requirements of the DNA recombinant material. Such media are well-known to those skilled in the art.

After fermentation, the cells can be removed from the fermentation broth by centrifugation or filtration. Depending on whether the host cells have secreted the GLA2 of the invention into the medium or whether the GLA2 are still connected to the host cells in some way either in the cytoplasm, in the periplasmic space or attached to or in the membrane or cell wall, the cells can undergo further treatment to obtain the recombinant protein. In the latter case, where the recombinant enzyme is still connected to the cells, recovery may be accomplished by rupturing the cells for example by high pressure, sonication, enzymatic digestion or simply by cell autolysis followed by subsequent isolation of the desired product. The GLA2 can be separated from the cell mass by various methods, such as ultrafiltration, and then subsequently precipitated with an organic solvent. The isolated GLA2 may be further purified by conventional methods such as precipitation and/or chromatography.

The present invention also relates to the use of the purified GLA2 or the above mentioned cells to hydrolyse carbohydrate-containing materials, such as mixtures of a source of proteins and a source of carbohydrates, especially of a mixture of a leguminous plant or of a cooked oleaginous plant and of a cooked or roasted cereal source, for example of a mixture of soya or cooked beans and of cooked or roasted wheat or rice.

DNA manipulation, cloning and transformation of bacteria cells are, except where otherwise stated, carried out according to the textbook of Sambrook *et al*. (Sambrook *et al*., Molecular Cloning, A Laboratory Manual, Cold Spring Harbor Laboratory Press, U.S.A., 1989). These examples are preceded by a brief description of the figures. The strains *Endomyces fibuliger* 8014, *Saccharomyces cerevisiae* YP107/pDP514, *Aspergillus oryzae* TK3 were deposited under the Budapest Treaty, at the Collection Nationale de Culture de Microorganismes (C.N.C.M.), 25 rue du docteur Roux, 75724 Paris, France, in March 17^{th}, 1998, *(E. fibuliger* and *S. cerevisiae*) and June 24^{th}, 1997, respectively, where they receive the deposit number CNCM I-1991, CNCM I-1992 and CNCM I-1882. All restrictions as to the availability of these deposits will be withdrawn upon first publication of this application or another application which claims benefit of priority to this application.

### Brief description of the figures

- Figure 1 shows the plasmid pDP514 used to clone *GLA2* in *Saccharomyces cerevisiae*
- Figure 2 shows the plasmid pDP490 used to complement the multiple deletions of the strain YP107.

### Example 1 Cloning of GLA2 by PCR

Chromosomal DNA was prepared from *Endomyces fibuliger* strain 8014 vegetative cells, also called *Sacchromycopsis fibuliger*, as described previously (Nga *et al*., World Journal of Microbiology and Biotechnology, 10, 465-471, 1994). This DNA was amplified with the oligonucleotides SEQ ID NO:3 and SEQ ID NO:4 for 30 cycles of 95°C for 30 seconds, 40°C for 30 seconds and 72°C for 2 minutes in a Perkin-Elmer DNA Thermal Cycler. The product was electrophoresed and the 470 bp amplicon cut out of the gel and purified using the QIAGEN QIAquick Gel Extraction Kit (Cat. No. 28704) according to the instructions. The amplicon was cloned using the Promega pGEM®-T vector (Cat. No. A3600) and electro-transformed into electro-competent *E. coli* strain BZ234. A plasmid was sequenced using the dideoxy chain termination method with Amersham Thermo Sequenase fluorescent labelled primer cycle sequencing kit (Cat. No. RPN2536) and the pUC19 sequencing primers labelled with the fluorescent dye IRD-41 (MWG Biotech). The reactions were analysed using the Li-cor dNA sequencer model 4000L and compiled with the GCG suite of programs (Devereux *et al*., Nucleic Acids Res., 12, 387 - 395, 1984).

The results are presented in the sequence listing below. The DNA sequence SEQ ID NO: showed no significant similarity to the *E. fibuliger GLU1* gene (Itoh *et al.,* J. Bact., 169, 4171-4176, 1987), but revealed a high degree of similarity to the *Schwanniomyces occidentalis* glucoamylase gene, *GAM1* (Strasser *et al.,* Eur. J. Biochem., 184, 699-706, 1989). This new glucoamylase from *E. fibuliger* was named *GLA2.*

To find the appropriate genomic sequence of *GLA2*, the following assays were performed. Accordingly, the *E. fibuliger* 8014 chromosomal DNA was digested with selected restriction enzymes, the fragments separated according to size by electrophoresis and transferred to a nylon membrane by capillary action. The above PCR amplicon was radioactively labelled using the *redi*prime random primer labelling kit from Amersham (Cat. No. RPN1633) and the nylon filter hybridised according to instructions. After washing and exposure, a 10 kb *Eco*RI restriction fragment was identified for cloning. Ten µg of chromosomal DNA was digested with the restriction enzyme *Eco*RI and the fragments separated according to size by electrophoresis. The region containing the 10 kb band size (8-12 kb) was cut out of the gel and the DNA purified using the QIAGEN QIAquick Gel Extraction Kit. This pool of fragments was cloned into the *E. coli* vector pK19 (Pridmore *et al*., Gene, 56, 309-312, 1987) previously digested with the restriction enzyme *Eco*RI and dephosphorylated. The ligation was electro-transformed into the *E. coli* strain BZ234 and the transformants selected on LB plates supplemented with 50 µg/ml kanamycin, 150 µg/ml 5-bromo-4-chloro-3-indolyl-β-D-galactotoside (Xgal) and 30 µg/ml isopropyl-β-thiogalactopyranoside (IPTG) and incubated at 37°C overnight. White colonies containing inserts were picked into microtiter wells containing 150 µl of LB medium supplemented with 50 µg/ml kanamycin and incubated at 37°C to form mini cultures. Aliquots from the 12-micotiter wells in the rows were pooled, a 1 µl sample taken for a PCR reaction with the PCR oligonucleotides described above to identify the microtiter row containing a positive clone. The PCR was repeated on the individual wells from the positive row to identify a clone carrying the *E. fibuliger GLA2* gene and named pDP472.

A restriction map of the plasmid pDP472 was prepared using standard techniques. From this a selection of single and double restriction digestions were performed, the resulting fragments separated according to size by electrophoresis and transferred to a nylon membrane. This membrane was hybridised with the radioactively labelled *GLA2* PCR product to localise the *GLA2* gene to a 4.0 kb *Eco*RI - *Hpa*I restriction fragment within the 10 kb *Eco*RI fragment of pDP472. This 4.0 kb *Eco*RI-*Hpa*I restriction fragment was subcloned into pUC19 (Yanisch-Perron *et al*., Gene, 33, 103-119, 1985) to give the clone pUC19-Sf-GLA2 and the DNA insert sequenced in its entirety on both strands. The DNA sequence shows the presence of one translation product of 963 amino acids with a predicted molecular weight of 107,473 Dalton. The *E. fibuliger* GLA2 protein shows no similarity to the *E. fibuliger* GLU1 or GLA1 protein, but does show a significant 82.5% similarity (71.5% identity) to the *S. occidentalis* GAM1 protein. The DNA sequence and the translation of the *E. fibuliger* GLA2 protein are shown in the listing of sequences below (see SEQ ID NO:1 and NO:2).

### Example 2 Expression of the Endomyces fibuliger GLA2 gene in Saccharomyces cerevisiae.

For the expression of the *E. fibuliger GLA2* gene in *S. cerevisiae* we chose to use the strong galactose inducible *S. cerevisiae GAL1* promoter for the following reasons: i) the native *E. fibuliger* promoter is not active (or not strong enough) in *S. cerevisiae* (data not shown) and ii) that by growing the yeast on galactose we would not interfere with future glucose assays from starch degradation.

The *E. fibuliger GLA2* coding region was PCR amplified from plasmid pDP472 with the oligonucleotides SEQ ID NO:5 and SEQ ID NO:6 using a high fidelity *Pwo* DNA polymerase from Boehringer Mannheim (Cat. No. 1644947) using the amplification conditions of 95°C for 30 secconds, 40°C for 30 seconds, 72°C for 3 minutes and repeated for 30 cycles. The PCR product was purified using the QIAquick PCR Purification kit (Cat. No. 28104), an aliquot digested with the restriction enzyme *Bam*HI and the fragment electrophoresed on an agarose gel. The corresponding 2.9 kb fragment was cut out of the gel and the DNA purified using the QIAquick Gel Extraction Kit. This fragment was then ligated into the *E.coli* plasmid pUC19 previously digested with the restriction enzyme *Bam*HI and dephosphorylated. A positive insert-containing clone was sequenced in its entirety to confirm the fidelity of the amplification.

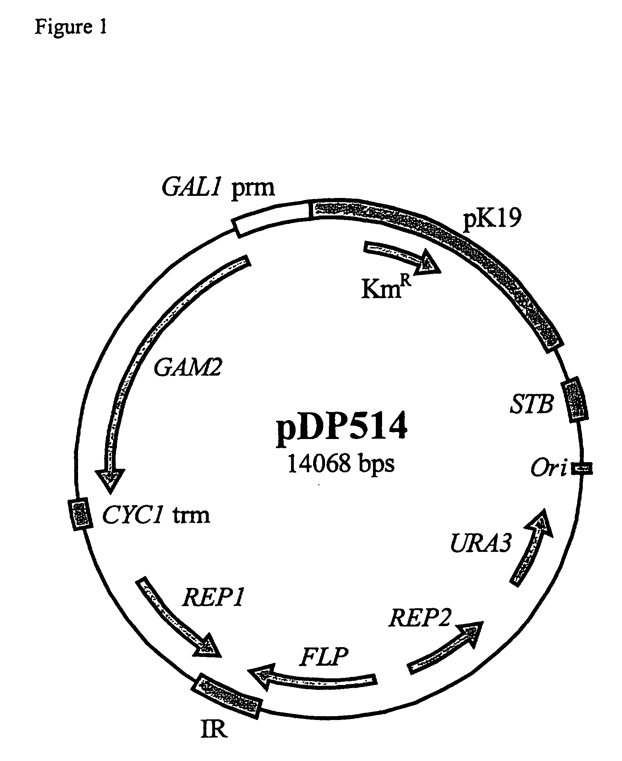

The yeast expression vector pDP509 was used. This plasmid consists of the *E.coli* vector pK19 conferring kanamycin resistance and replication for construction and plasmid amplification in *E. coli.* pDP509 contains the yeast *Saccharomyces cerevisiae* natural 2 micron plasmid (Hartley *et al.,* Nature, 286, 860-864, 1980) for high stability, high/low copy number replication in this host and the *URA3* (Rose *et al*., Gene, 29, 113-124, 1984) gene for selection of the plasmid in the appropriate host. Genes to be expressed are cloned into either the *Bam*HI or *Xho*I restriction sites, where expression is under control of the yeast *GAL1* galactose inducible promoter (Johnston *et al.,* Mol. Cell. Biol., 4, 1440-1448, 1984) and the yeast *CYC1* transcription terminator (Osborne *et al*., Proc. Natl. Acad. Sci. U.S.A., 86, 4097-4101, 1989). Plasmid pDP509 was digested with the restriction enzyme *Bam*HI plus dephosphorylated and ligated with the *E. fibuliger GLA2* gene *Bam*HI fragment. The resulting plasmid was named pDP514 and is shown in Figure 1 [according to the following method, this plasmid is also available from the transformed *S. cerevisiae* YP107 strain (YP107/pDP514) which has been deposited at the C.N.C.M. under the number CNCM I-1992].

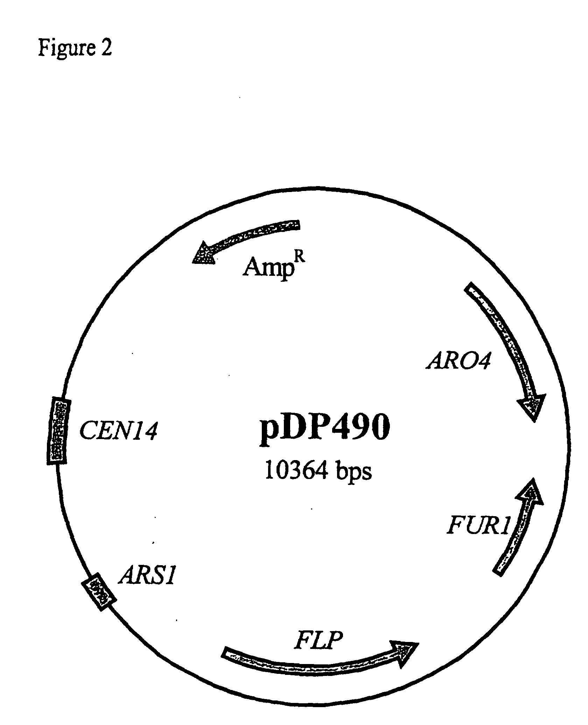

The *S. cerevisiae* YP107 expression host is a diploid Baker's yeast strain from India. This strain was cured for its native 2 micron plasmid and the host *URA3, FUR1* (Kern *et al*., Gene, 88, 149-157, 1990) and *URK1* (Kern *et al*., Nucleic Acids Res., 18, 5279-5283, 1990) genes stepwise disrupted. This strain is incapable of synthesising uracil, or of utilising either uridine or cystidine from the growth medium (Napp *et al.,* Biotechnology and Bioengineering, 41, 801-810, 1993). The multiple deletions of this strain were complemented by the plasmid pDP490 (see Figure 2), a yeast centromere plasmid carrying the yeast *FUR1* gene, allowing the strain to utilise uracil supplemented in the growth medium. This strain, named YP107, can also ferment galactose efficiently. Strain YP107 was electro-transformed (Gysler *et al*., Biotechnology Techniques, 4, 285-290, 1990) with the plasmid pDP514 and the transformants selected on minimal medium plates (selecting for the plasmid *URA3* gene and synthesis of uracil by the host). These transformants were then replica streaked on the same minimal medium plates containing 5-flourouracil (Sigma Cat. No. F-6627), a compound that lethally selects against *FUR1* positive strains, hence against these yeast carrying pDP490. The final result is the yeast strain YP107 transformed with plasmid pDP514 that is stable under any growth conditions (strain YP107/pDP514).

The yeast strains YP107/pDP514 were patched onto agar plates containing 0.67% yeast nitrogen base (Difco, Cat. No. 0919-15-3), 2% D-galactose (Difco, Cat. No. 0163-17-4) and 3% soluble starch (Sigma, Cat. No. S-9765). After incubation at 30°C for several days, visible faint halos were seen surrounding the areas of yeast growth.

The glucoamylase activity was then assayed in culture supernatant of the *S. cerevisiae* transformants that produced turbid precipitates in starch-containing plates. The assay is performed by reacting culture filtrate with soluble starch and detecting the glucose liberated using commercially-available kits.

### Example 3 Purification of the Endomyces fibuliger GLA2 recombinant protein produced by Saccharomyces cerevisiae.

The yeast YP107/pDP514 of example 2 is grown in 1 l galactose minimal medium (6.7 g bacto-yeast nitrogen base, 20 g galactose) with areation by batch culture in a fermentor or in shake flasks at 30°C until a strong culture has developed. The yeast cells are removed by centrifugation or filtration and the culture medium loaded onto a Separose Q 16/200 column. The column is washed with 5 column volumes of 20 mM Tris HCl, pH 8.0 and the proteins then eluted in the same buffer with a NaCl gradient from 0 to 1 M over 10 column volumes. The fractions are assayed by incubating a 100 µl sample with 1% soluble starch in phosphate buffer pH 8.0 for 1 hour at 30 °C and the release of glucose measured with the TC D-glucose test kit from Boehringer Mannhein according to the included instructions. The active fractions are pooled and dialysed against 20 mM Tris HCI, pH 8.0. The sample is then loaded onto a Superose Q 16/200 column in 20 mM Tris HCl pH 8.0 and then eluted in the same buffer with a NaCl gradient from 0 to 1 M over 20 column volumes. The active fractions are identified as described above and pooled. Solid ammonium sulfate is added to a final concentration of 1 M and the sample is then loaded onto a phenyl sepherose 10/10 column and eluted in 50 mM phosphate buffer pH 7.0 and a gradient from 1 to 0 M (NH₄)₂SO₄ over 10 column volumes. The active fractions are identified, pooled and concentrated. The sample is finally loaded on a superose 6 16/500 column and eluted with 50 mM phosphate buffer pH 7.0 plus 200 mM KCl. The active fractions are identified, pooled and stored frozen at -20°C.

### Example 4 Expression of the Endomyces fibuliger GLA2 gene in Aspergillus oryzae

Expression of the *Endomyces fibuliger GLA2* gene in *Aspergillus oryzae* was achieved by amplification of the coding region of the *GLA2* gene from *Endomyces fibuliger* and cloning of the PCR product into the expression vector pFBY182. The plasmid pFBY182, containing the *pep*B gene as a *Eco*RI-*Xba*I fragment under the control of the *Aspergillus niger pki*A promoter and terminator was obtained from Novartis, Switzerland, via Dr. Gabor Jarai. The nucleotide sequence of pFBY182 is available in the GenBank accession number S38698.

The *E. fibuliger GLA2* coding region was PCR amplified from plasmid pDP472 with the oligonucleotides SEQ ID NO:7 and SEQ ID NO:8 using a high fidelity *Pwo* DNA polymerase from Boehringer Mannheim (Cat. No. 1644947) using the amplification conditions of 95°C for 30 secconds, 40°C for 30 seconds, 72°C for 3 minutes and repeated for 30 cycles. The PCR product was purified using the QIAquick PCR Purification kit (Cat. No. 28104), an aliquot digested with the restriction enzymes *Eco*RI and *Xba*I (*Eco*RI site in 5' end and a *Xba*I site in the 3' end) thus allowing directional cloning into *Eco*RI-*Xba*I digested fungal expression vector pFBY182, and the fragment electrophoresed on an agarose gel.

The corresponding 2.9 kb fragment was cut out of the gel and the DNA purified using the QIAquick Gel Extraction Kit. This fragment was then ligated into the pFBY182 plasmid previously digested with the restriction enzyme restriction enzymes *Eco*RI and *Xba*I and dephosphorylated.

In the resulting plasmid pDP800, *GLA2* transcription is under control of the *A. niger pki*A promoter and terminator (Graaff, Curr. Genet., 22, 21-27, 1992).

pDP800 was then introduced into *A. oryzae* NF1 (*pyr*G1) by co-transformation with pNFF28 containing the *A. oryzae pyr*G gene. *Aspergillus oryzae* NF1 (*pyr*G1) is a uridine auxotroph derivative of *A. oryzae* TK3 in which the *pyr*G gene, encoding orotidine 5'-phosphate decarboxylase, was inactivated by targeted disruption. Plasmid pNFF28 contains the *A. oryzae* TK3 *pyr*G gene. The nucleotide sequence of pNFF28 is available in the GenBank accession number Y13811.

Accordingly, *A. oryzae* NF1 was grown in MM with 0.1% yeast extract, 50 mM glucose and 5 mM glutamine (MM contains per litre 1.5 g KH₂PO₄, 0.5 g MgSO₄.7H₂O, 0.5 g KCl). The mycelium was harvested by sterile filtration, washed once with sterile double distilled water and once with K0.8MC (20 mM MES-HCl pH 5.8, 0.8 M KCl, 50 mM CaCl₂). 1.5 g of mycelium was resuspended in 20 ml of a filter sterilized 5 mg/ml solution of Novozyme 234 in K0.8MC. The mycelium suspension was incubated at 30°C for 2 hours with gentle agitation (120 rpm). The protoplasts were liberated from the mycelium by gentle resuspension with a pipet, washed twice with 20 ml of S1.0TC (10 mM Tris-HCl pH 7.5, 1 M Sorbitol, 50 mM CaCl₂) and were resuspended in a final concentration of 10⁸/ml in S1.0TC. 20 µl of DNA was mixed with 200 µl of protoplasts and 50 µl of 25% PEG 6000 in 10 mM Tris-HCl pH 7.5, 50 mM CaCl₂ and incubated for 20 min on ice. To this mixture, 2 ml of 25% PEG 6000 (BDH) in 10 mM Tris-HCl pH 7.5, 50 mM CaCl₂ were added, gently mixed and incubated for 5 min at room temperature. 4 ml of S1.0TC was added and 1.0 ml aliquots were mixed with 5 ml of 2% low melting point agarose (Sigma) in OFNB (osmotically stabilized fungal nitrogen base) and plated onto OFNB agar (Difco) with 50 mM glucose and 10 mM NaNO₃. *A. oryzae* NF1 transformants were plated on MM agar with 1 M sucrose, 50 mM glucose and 5 mM glutamine.

The resulting transformants were screened on minimal plates (URA3 selection). Among all transformants, strain B exhibits high degree of hydrolysing specificities against starch containing materials.

### Example 5 Functional derivatives of the GLA2

Functional derivatives of the recombinant glucoamylase GLA2 of *Endomyces fibuliger* (SEQ ID NO:2) are prepared according to a method adapted from the method described by Adams *et al*. (EP402450; Genencor). Briefly, the expression cassette pDP514 containing the *GLA2* gene (SEQ ID NO:1) was subjected to an *in-vitro* chemical mutagenesis by hydroxylamine. According to example 2, the mutagenised DNA was then used to transform *S. cerevisiae* YP107. The transformants were patched onto agar plates containing 0.67% yeast nitrogen base (Difco, Cat. No. 0919-15-3), 2% D-galactose (Difco, Cat. No. 0163-17-4) and 3% soluble starch (Sigma, Cat. No. S-9765). After incubation at 30°C for several days, visible faint halos were seen surrounding the areas of yeast growth. Functional derivatives of the GLA2 were finaly detected by comparing the size of the halos to the one produced by YP107/pDP514 (the reference). Functional derivatives presenting bigger halos than the reference were selected. DNA sequencing analysis revealed modifications in the original *GLA2* gene, such as a deletion, addition and/or a substitution of some amino acids. Amino acid identities of the GLA2 derivatives over the original GLA2 are superior to 90%.

### Examples 6

For preparing a fermented soya sauce the *Aspergillus oryzae* strain B of example 4 was used. Accordingly, a koji is prepared by mixing the *Aspergillus oryzae* strain B, previously grown as a koji culture, with a mixture of cooked soya and roasted wheat and rice, the koji is then hydrolysed in aqueous suspension for 3 to 8 hours at 45°C to 60°C with the enzymes produced during fermentation of the *Aspergillus oryzae* culture, a moromi is further prepared by adding suitable amount of sodium chloride to the hydrolysed koji suspension, the moromi is left to ferment and is then pressed and the liquor obtained is pasteurized and clarified.

### Examples 7

For producing a flavouring agen sauce the *Aspergillus oryzae* strain B of example 4 was used. Accordingly, a aqueous suspension of a mixture of cooked soya and roasted wheat and rice is prepared, the proteins are solubilized by hydrolysis of the suspension with a protease at pH6.0 to 11.0, the suspension is heat-trated at pH 4.6 to 6.5, and the suspension is ripened with enzymes of a koji culture fermented by *Aspergillus oryzae* strain B.

## Claims

1. A recombinant glucoamylase from *Endomyces fibuliger* comprising the amino acid sequence starting from one of the amino acids 1 - 50 up to amino acid 963 of SEQ ID NO:2, or functional derivatives thereof having at least 85% identity.

2. A recombinant glucoamylase according to claim 1 which is fused to a leader peptide.

3. A recombinant glucoamylase according to claim 2 which is fused to the leader peptide of *Endomyces fibuliger* having the amino acid sequence starting from amino acid 1 to one of the amino acids 15 - 30 of SEQ ID NO:2, or functional derivatives thereof having at least 85% identity.

4. A leader peptide of *Endomyces fibuliger* having the amino acid sequence starting from amino acid 1 to one of the amino acids 15 - 30 of SEQ ID NO:2.

5. A DNA molecule which comprises a *GLA2* gene encoding the enzyme according to claim 1.

6. A DNA molecule according to claim 5, which is a vector comprising the *GLA2* gene.

7. A DNA molecule according to claim 5, wherein the *GLA2* gene is operably linked to at least one regulatory sequence able to direct the expression of the gene.

8. A DNA molecule according to claim 7, wherein the regulatory sequence is derived from another organism than the one from which the *GLA2* gene is derived

9. A DNA molecule according to claim 5, wherein the *GLA2* gene comprises the coding parts of the nucleotide sequence SEQ ID NO:1 or functional derivatives thereof due to the degeneracy of the genetic code

10. A cell which expresses the proteins according to claims 1-3 by recombinant technology

11. A cell according to claim 10, which is *Sacchromyces cerevisiae* or a koji mold.

12. A cell according to claim 11 which is the deposited cell CNCM I-1992.

13. A cell according to claim 10 which is able to over-express the enzyme.

14. A method for producing the enzyme according to claim 1, comprising cultivating recombinant cells according to claims 10-13 in a suitable growth medium under conditions that the cells express the enzyme, and optionally isolating the enzyme in the form of a concentrate

15. Use of the recombinant protein according to claim 1-3 or the cells according to claims 10-13 to hydrolyse carbohydrate-containing materials, preferably materials containing rice carbohydrates.

## Patentansprüche

1. Rekombinante Glucoamylase aus *Endomyces fibuliger*, das die bei einer der Aminosäuren 1-50 bis zur Aminosäure 963 der Seq-ID Nr.2 startende Aminosäure-Sequenz, oder funktionale Derivate davon mit zumindest 85 % Identität umfasst.

2. Rekombinante Glycoamylase nach Anspruch 1, die an ein Leit-Peptid geschmolzen ist.

3. Rekombinante Glycoamylase nach Anspruch 2, die an das Leit-Peptid von *Endomyces fibuliger* geschmolzen ist, das die bei der Aminosäure 1 bis zu einer der Aminosäuren 15-30 der Seq-ID Nr.2 startende Aminosäure-Sequenz oder funktionale Derivate davon mit zumindest 85 % Identität aufweist.

4. Leit-Peptid von *Endomyces fibuliger*, das die bei der Aminosäure 1 bis zu einer der Aminosäuren 15-30 der Seq-ID Nr.2 startende Aminosäure-Sequenz aufweist.

5. DNA-Molekül, das ein *GLA2*-Gen umfasst, das das Enzym nach Anspruch 1 kodiert.

6. DNA-Molekül nach Anspruch 5, das ein das *GLA2*-Gen umfassender Vektor ist.

7. DNA-Molekül nach Anspruch 5, worin das *GLA2*-Gen mit zumindest einer Regulationssequenz funktionsfähig verbunden ist, die die Expression des Gens leiten kann.

8. DNA-Molekül nach Anspruch 7, worin die Regulationssequenz aus einem anderen Organismus hergeleitet ist als dem, von dem das *GLA2* hergeleitet ist.

9. DNA-Molekül nach Anspruch 5, worin das *GLA2-Gen* die kodierenden Teile der Nucleotidsequenz Seq-ID Nr. 1 ode funktionale Derivate davon aufgrund der Degeneration des genetischen Codes umfasst.

10. Zelle, die die Proteine expremiert, nach den Ansprüchen 1-3 durch eine rekombinante Technologie.

11. Zelle nach Anspruch 10, die *Sacchromyces cerrevisiae* oder ein Koji-Pilz ist.

12. Zelle nach Anspruch 11, die die hinterlegte Zelle CNCM 1-1992 ist.

13. Zelle nach Anspruch 10, die das Enzym über-expremieren kann.

14. Verfahren zum Herstellen des Enzyms nach Anspruch 1, welche umfasst, Kultivieren rekombinanter Zellen nach den Ansprüchen 10-13 in einem geeigneten Wachstumsmedium unter Bedingungen, dass die Zellen das Enzym expremieren, und wahlweise Isolieren des Enzyms in der Form eines Konzentrats.

15. Verwendung des rekombinanten Proteins nach den Ansprüchen 1-3 oder der Zellen nach den Ansprüchen 10-13, um die Kohlenhydrat-enthaltenden Materialen, vorzugsweise Reis-Kohlenhydrate enthaltende Materialien, zu hydrolysieren.

## Revendications

1. Glucoamylase recombinante issue de *Endomyces fibuliger* comprenant la séquence d'acides aminés en partant d'un des acides aminés 1-50 jusqu'à l'acide aminé 963 de la SEQ ID NO:2, ou dérivés fonctionnels de celle-ci contenant au moins 85% d'identité.

2. Glucoamylase recombinante suivant la revendication 1, qui est fusionnée à un peptide leader.

3. Glucoamylase recombinante suivant la revendication 2, qui est fusionnée au peptide leader de *Endomyces fibuliger* comprenant la séquence d'acides aminés en partant de l'acide aminé 1 à un des acides aminés 15-30 de la SEQ ID NO:2, ou dérivés fonctionnels de celle-ci contenant au moins 85% d'identité.

4. Peptide leader de *Endomyces fibuliger* comprenant la séquence d'acides aminés en partant de l'acide aminé 1 à un des acides aminés 15-30 de la SEQ ID NO:2.

5. Molécule d'ADN qui comprend un gène *GLA2* codant pour l'enzyme suivant la revendication 1.

6. Molécule d'ADN suivant la revendication 5, qui est un vecteur comprenant le gène *GLA2*.

7. Molécule d'ADN suivant la revendication 5, dans laquelle le gène *GLA2* est lié de manière fonctionnelle à au moins une séquence régulatrice capable de diriger l'expression du gène.

8. Molécule d'ADN suivant la revendication 7, dans laquelle la séquence régulatrice est dérivée d'un organisme autre que celui dont le gène *GLA2* est dérivé.

9. Molécule d'ADN suivant la revendication 5, dans laquelle le gène *GLA2* comprend les parties codantes de la séquence de nucléotides SEQ ID NO:1 ou de dérivés fonctionnels de celle-ci dus à la dégénérescence du code génétique.

10. Cellule qui exprime les protéines suivant les revendications 1-3 par une technologie de recombinaison.

11. Cellule suivant la revendication 10, qui est une *Sacchromyces cerevisiae* ou un champignon de type Koji.

12. Cellule suivant la revendication 11, qui est la cellule déposée CNCM I-1992.

13. Cellule suivant la revendication 10, qui est capable de sur-exprimer l'enzyme.

14. Procédé pour la production de l'enzyme suivant la revendication 1, comprenant la culture de cellules recombinantes suivant les revendications 10-13 dans un milieu de croissance approprié dans des conditions telles que les cellules expriment l'enzyme, et éventuellement l'isolement de l'enzyme sous la forme d'un concentré.

15. Utilisation de la protéine recombinante suivant les revendications 1-3 ou des cellules suivant les revendications 10-13 pour hydrolyser des matières contenant des hydrates de carbone, de préférence des matières contenant des hydrates de carbone du riz.
